# NEW EUROPEAN PATENT SPECIFICATION

(11) **EP 0 236 002 B2**
(45) Date of publication and mention of the opposition decision: **23.10.1996**
(45) Mention of the grant of the patent: 03.02.1993
(21) Application number: 87301335.3
(22) Date of filing: 17.02.1987
(51) Int. Cl.: A61K 9/22

(54) **Pharmaceutical composition comprising a medicament and a water-swellable polymer**
Einen Wirkstoff und ein in Wasser quellendes Polymer enthaltendes Arzneimittel
Composition pharmaceutique contenant un médicament et un polymère gonflant dans l'eau

(30) Priority: 18.02.1986 GB 8603964
(43) Date of publication of application: 09.09.1987
(73) Proprietor: MALLINCKRODT VETERINARY, INC., Lake Forrest, IL 60045 (US)
(72) Inventor: Baker, Rodney Cyril The Wellcome Research Lab., Berkhamsted Hertfordshire (GB)
(74) Representative: Bassett, Richard Simon

(56) References cited:
- EP-A- 0 051 879
- EP-A- 0 157 695
- EP-A- 0 223 590
- EP-B- 0 165 577
- DE-A- 3 309 516
- FR-A- 2 260 993
- GB-A- 2 057 262
- US-A- 4 066 754
- CHEMICAL ABSTRACTS, vol. 80, no. 1, 14th January 1974, page 239, abstract no. 6882n, Columbus, Ohio, US; S.K. BAVEJA et al.: "Effect of formulation factors on the dissolution rate of sulfadimidine tablets", & INDIAN J. TECHNOL. 1973, 11(3), 137-8
- CHEMICAL ABSTRACTS, vol. 89, no. 2, 10th July 1978, page 367, abstract no. 12090e, Columbus, Ohio, US; T.N. ZAKRZHEVSKAYA et al.: "Possibility of using a new excipient in barbital and sodium barbital tablets", & FARMATSIYA (MOSCOW) 1978, 27(2), 19-23
- CHEMICAL ABSTRACTS, vol. 98, no. 24, June 1983, page 363, abstract no. 204309r, Columbus, Ohio, US; M. NAKANO et al.: "Sustained release of theophylline from hydroxypropyl cellulose tablets", & J. PHARM. SCI. 1983, 72(4), 378-80
- Dow, "Methocel as a binding agent for tablet production by wet granulation", 1985

## Description

The present invention relates to novel compositions and unit dose forms for release of biologically active substances into desired environments.

It has been known in the field of human medicine to provide pharmaceutical compositions for release of drug over a prolonged period by incorporating a water swellable polymer, such as a cellulose ether, therein. In the stomach, the polymer is believed to form a gel layer over the exterior surface of the pharmaceutical dose unit (for example a tablet) so that the drug then diffuses through this layer. Previously, it has been found that generally, the more polymer in the composition, the slower is the effective rate of drug release. For this reason, the overall polymer content of such compositions has tended to be a substantial porportion by weight of the total.

British Patent No 1,330,829 and United States Patent No 3,507,952 disclose sustained release dosage forms for veterinary use in which the sustained release pattern is achieved by the inclusion of a certain proportion of a lubricant. US Patent No 3,773,921 describes sustained release dosage forms for oral administration to ruminant animals, which comprise a therapeutically active sulfa compound, dibasic calcium phosphate, a binder and a lubricant. These patent specifications however make no reference to the use of water swellable polymers such as cellulose ethers.

The present applicants have now found that in compositions for oral administration to the rumen of an animal, or for other purposes in which the composition is exposed to like conditions, it is advantageous to include a water swellable polymer. This is not necessarily for the purpose of providing slow release but can be for other reasons, such as inhibiting disintegration of the dose unit prior to use. This is particularly the case when the dose unit is in the form of a bolus of compressed powder or granules. The applicants have surprisingly further found that it is advantageous for the total percentage polymer content to be less than that in previously known compositions, particularly if a slow release profile is desired. The applicants believe that in such circumstances, the rate of drug release may actually decrease (i.e. the duration of release is prolonged) with decreasing polymer content, which is the opposite to what has been observed with the prior compositions referred to above.

Thus, one aspect of the present invention provides a veterinary composition for release of a biologically active substance into the rumen of an animal, which composition comprises said biologically active substance and a water swellable polymer material, characterised in that the water swellable polymer material is hydroxypropylmethylcellulose and constitutes from 0.01% to 4% by weight of the said composition.

A unit dose form according to the present invention may be for example a pharmaceutical unit dose for oral administration to an animal such as a bolus, tablet, cachet or lozenge.

Especially preferred dosage forms for administration to ruminant animals (e.g. cattle and sheep) are boluses wherein the ingredients are present in the form of compressed powders or granules. To inhibit regurgitation of an intra-rumenal bolus, the bolus should be provided with retention means, for example a geometric feature such as wings (for example as described in UK Patent Specification No: 2 020 181). Alternatively, the retention means may provide overall sufficient density to prevent regurgitation. This may be a weighted metal tube surrounding the exterior of the bolus, such as described in UK Patent Specification No: 1 603 970, a discrete weight such as described in European Patent Specification No: EP 0 164 927 A2 or a densification ingredient mixed with the composition. A favourable material for this purpose is iron powder. Others include iron filings, other dense metals such as tungsten and dense inorganic compounds, for example barium sulphate, calcium sulphate and calcium phosphate.

Biologically active substances which may be administered to animals in a pharmaceutical composition according to the present invention include pharmacologically active agents such as anti-infectives, eg. antibacterials and anthelmintics; animal growth promoters; and animal nutrients. Other biologically active substances which may be administered to animals include insecticides and larvicides. In general the biologically active substance may be any of those described in European Patent Specification No. 164 927.

Preferred pharmacological agents for veterinary use include anti-infective agents such as anthelmintics and antibacterials.

Preferred antibacterials include sulphonamides and salts thereof (e.g. sulfanilamide, sulfadiazine, sulfamethisazole, sulfapyridine, sulfathiazole, sulfamerazine, sulfamethazine, sulfisoxazole, sulformethoxine, 2-(p-aminobenzene)-sulfonamide-3-methoxypyrazine (Kelfizina), sulfonyldianiline, mafenide, 5-sulfanilamido-2,4-dimethylpyrimidine, 4-(N'-acetylsulfanilamide)-5,6-dimethoxypyrimidine, 3-sulfanilamido-4,5-dimethylisoxazole, 4-sulfanilamido-5-methoxy-6-decyloxypyrimidinesulfamonomethoxine, 4-p-(8-hydroxyquinolinyl-4-azo)phenylsulfanilamido-5,6-dimethoxypyrimidine, sulfadimethoxine, sulfadimidine, sulfamethoxazole, sulfamoxole, sulfadoxine, sulfaguanidine, sulfathiodimethoxine, sulfaquinoxaline, and p-(2-methyl- 8-hydroxyquinolinyl-5-azo)-phenylsulfanilamido-5,6-dimethoxypyrimidine); and 2,4-diaminopyrimidines and salts thereof (eg. 2,4-diamino-6-ethyl-5-p-chlorophenylpyrimidine (Pyrimethamine), 2,4-diamino-5-(3',4',5'-trimethoxybenzyl)pyrimidine (Trimethoprim),2,4-diamino-5-(3',4'-dimethoxybenzyl)pyrimidine (Diaveridine), 2,4-diamino-5-(2'-isopropyl-4'-chlorophenoxy) pyrimidine, 2,4-diamino-5-methyl-6-sec-butyl-pyrido (2,3-d) pyrimidine, 2,4-diamino-5-methyl-6-benzylpyrido (2,3-d)pyrimidine, 2,4-diamino-6-benzylpyrido (2,3-d) pyrimidine, 2,4-diamino-5,6-trimethylenequinazoline, 2,4-diamino-5,6-tetramethylenequinazoline, 2,4-diamino-5-(4'-dimethylamino-3',5'-dimethoxybenzyl)pyrimidine 2,4-diamino-5-(2',4',5'-trimethoxybenzyl) pyrimidine,2,4-diamino-5-(2'-ethyl-4',5'-dimethoxybenzyl)pyrimidine, and 2,4-diamino-5-(2'-methyl-4',5'-dimethoxybenzyl) pyrimidine (Ormetoprim), as well as the 2,4-diaminopyrimidine derivatives disclosed in European Patent No. 51879). A particularly preferred 2,4-diaminopyrimidine for use in pharmaceutical compositions according to the present invention is 2,4-diamino-5-(8-dimethylamino-7-methyl-5-quinolylmethyl)pyrimidine or a salt thereof. A particularly preferred sulphonamide is sulphadimidine or a salt thereof.

Preferred anthelmintics include levamisole, tetramisole, oxfendazole, mebendazole, fenbendazole, thiabendazole, albendazole and ivermectin.

Water-swellable HPMC polymers which may be utilised in the composition of the present invention are generally those having a number average molecular weight in the range 10,000 to 150,000. Preferably the water swellable polymer is HPMC K15M, HPMC K100M and HPMC E4M.

The water swellable polymer is preferably present in the composition at a % weight concentration of between 0.1 to 4.0%, especially 0.5 to 2.5%.

Preferred compositions according to present invention are those comprising a biologically active substance and a water swellable polymer material characterised in that the water swellable polymer material constitutes no more than 2% by weight of said composition.

As indicated hereinabove, applicants have found that decreasing the amount of polymer in the compositions according to the present invention can prolong the duration of release of the active ingredient. Thus, a sustained release formulation according to the present invention preferably contains no more than 1% by weight of polymer, for example between 0.1 and 1% by weight of polymer.

To achieve a particular release profile, eg. an immediate release followed immediately afterwards, or after an interval, by a slower prolonged release, it may be desirable to provide a mixture of two or more different formulations, which may for example be in the form of granules, each containing a different percentage of polymer.

Accessory ingredients which may be incorporated in a pharmaceutical composition according to the present invention are well known to those skilled in the art of human and veterinary pharmacy. Thus, accessory ingredients may include densification agents as described above; dense, water insoluble fillers (bulking agents) such as dibasic calcium phosphate, barium sulphate, or other fillers as described in US Patent Specification no 3, 773, 921; binders, such as polyvinylpyrolidone, gelatin, casein, acacia, tragacanth, agar and pectin; and lubricants such as magnesium stearate, sodium stearate, calcium stearate, stearic acid, talc or silica. If desired the formulations may also contain pigments such as red or yellow iron oxide.

It will be appreciated that the type and amounts of accessory ingredients may be varied depending on the precise formulation required.

Densification agents will generally be present at concentrations of from 5 to 75% by weight of the total composition, preferably 15 to 50%. Fillers will generally comprise 0 to 95% of the total composition. Binding agents are preferably present in an amount ranging from 1 to 10% and lubricants in the range 0.1 to 2%.

Pharmaceutical dosage forms according to the present invention may be prepared by techniques known to those skilled in the art of human and veterinary pharmacy. Thus, for example they may be prepared by direct compression of the admixed ingredients. Alternatively, the ingredients may first be granulated and the granules compressed. When the dosage form is prepared by granulation it may be convenient to add a lubricant e.g. magnesium stearate after the granulation step. As will be understood by those skilled in the art, the degree of compression will affect the hardness of the dosage form. It is preferred that, when measured by the method described hereinafter, the hardness of dosage forms according to the present invention lies in the range 100 to 18000 Newtons, preferably 100 to 1000 N for a short-acting unit dose form and preferably 800 to 1800N for a long-acting unit dose form. It will be appreciated however that the optimum hardness and the degree of compression required for a given dosage form can readily be determined by routine tests.

It will be appreciated that whilst the amount of polymer present is a critical factor in determining the release rate of the active ingredient, the precise duration and pattern of release will be affected to some extent by other factors such as the nature of the active ingredient, the degree of compression used in manufacturing the dosage form and its overall size. In general a unit dosage form according to the present invention may provide release of the active ingredient for up to 10 days. Thus for example a short acting unit dose form may provide continuous release of the active ingredient over a period of 1 to 96 hours eg. 2 to 48 hours from the time of administration, and a long acting dosage form may release the active ingredient during a period of 3 hours to 10 days eg. 12 hours to 7 days, from the time of administration.

In a preferred embodiment the present invention provides a short-acting veterinary bolus for oral administration to the rumen of an animal said bolus comprising one or more active ingredients, preferably an antibacterial agent such as 2,4-diamino-5-(8-dimethylamino-7-methyl-5-quinolylmethyl)pyrimidine or a salt thereof and/or a sulphonamide or a salt thereof, preferably sulphadimidine, together with a water swellable polymer, preferably hydroxypropylmethylcellulose, the polymer being at a % weight concentration of no more than 2%, preferably between 1 and 2%, and optionally one or more accessory ingredients such as a binder, lubricant, densification agent or pigment. In this embodiment the bolus typically contains from 1 to 8mg/kg of the pyrimidine, preferably approximately 4mg/kg; and from 9 to 72mg/kg of sulphadimidine, preferably approximately 36mg/kg. The unit dose form conveniently has a total weight of from 5 to 50g. The bolus preferably has a hardness (when measured by the method described hereinafter) of from 100 to 1000 Newtons, for example 140 to 180N for a 15g bolus and 300 to 900N for a 30g bolus. The length of the bolus is conveniently in the range 30 to 70mm preferably approximately 50mm, and its thickness is within the range 10 to 30mm, e.g. 12 to 13.5mm for a 15g bolus and 23 to 25mm for a 30g bolus.

In another preferred embodiment the present invention provides a long-acting veterinary bolus for administration to the rumen of an animal said bolus comprising one or more active ingredients, preferably an antibacterial agent such as 2,4-diamino-5-(8-dimethylamino-7-methyl-5-quinolylmethyl)pyrimidine or a salt thereof and/or a sulphonamide or a salt thereof, preferably sulphadimidine, together with a water swellable polymer, preferably hydroxypropylmethylcellulose, the polymer being at a % weight concentration of no more than 1%, preferably between 0.1 and 1%, and optionally one or more accessory ingredients such as a binder, lubricant, densification agent or pigment. In this embodiment the bolus typically contains from 6 to 16mg/kg of the pyrimidine, preferably approximately 8mg/kg; and from 54 to 144mg/kg of sulphadimidine, preferably approximately 72 mg/kg. The unit dose form conveniently has a total weight of from 5 to 50g. The bolus preferably has a hardness of from 800 to 1800 Newtons, for example 900 to 1500N for a 30g bolus. The length of the bolus is conveniently the range 30 to 70mm, preferably approximately 50mm, and its thickness is within the range 20 to 25mm, e.g. 21 to 23mm for a 30g bolus.

The present invention will now be illustrated by way of the following examples. Compound A is 2,4-diamino-5-(8-dimethylamino-7-methoxy-5-quinolylmethyl)pyrimidine. Compound B is 2,4-diamino-5-(8-dimethylamino-7-methyl-5-quinolylmethyl)pyrimidine

### Examples

### 1. Compressed Granule Bolus (Short Acting)

| Ingredient | % Content by Weight |
|---|---|
| Sulphadimidine | 47.97 |
| Compound A or Compound B | 5.33 |
| Iron Powder (325 Mesh) | 29.20 |
| Dibasic Calcium Phosphate | 10.00 |
| HPMC (Methocel K100M) | 2.00 |
| PVP K30 | 5.00 |
| Magnesium stearate | 0.50 |

All the ingredients apart from magnesium stearate were granulated. The magnesium stearate was added to the granulate and the mixture compressed using a Manesty 35T compressing machine to give boluses of the desired weight.

### 2. Compressed Granule Bolus (Long Acting)

| Ingredients | % Contents by Weight |
|---|---|
| Sulphadimidine | 47.97 |
| Compound A or Compound B | 5.33 |
| Iron Powder (325 Mesh) | 30.20 |
| Dibasic Calcium Phosphate | 10.00 |
| HPMC (Methocel K100M) | 1.00 |
| PVP K30 | 5.00 |
| Magnesium stearate | 0.50 |

The ingredients were granulated and compressed together to form a bolus, as described in Example 1.

### 3. Compressed Granule Bolus (Mixed Formulation)

| Ingredients | Formulation I % Content Weight | Formulation II % Content by Weight |
|---|---|---|
| Sulphadimidine | 47.97 | 47.97 |
| Compound A or Compound B | 5.33 | 5.33 |
| Iron Powder (325 Mesh) | 30.20 | 31.10 |
| Dibasic Calcium Phosphate | 10.00 | 10.00 |
| HPMC (Methocel K100M) | 1.00 | 0.10 |
| PVP K30 | 5.00 | 5.00 |
| Magnesium stearate | 0.50 | 0.50 |

Formulations I and II were granulated and intimately mixed together in a 1:1 ratio then compressed together to form a bolus, as described in Example 1.

### 4. Compressed Granule Bolus (Long Acting)

| Ingredients | % Contents by Weight |
|---|---|
| Sulphadimidine | 47.97 |
| Compound B | 5.33 |
| Iron Powder (325 Mesh) | 28.2 |
| Dibasic Calcium Phosphate | 10.00 |
| HPMC (Methocel K15M) | 1.00 |
| PVP K30 | 5.00 |
| Magnesium stearate | 0.50 |
| Red iron oxide | 2.00 |

The ingredients were granulated and compressed together to form a bolus as described in Example 1.

### Examples 5A - 5F

The following formulations were prepared using sulphadimidine as active ingredient:-

| | % content by wt. | | | | | |
|---|---|---|---|---|---|---|
| | 5A | 5B | 5C | 5D | 5E | 5F |
| Sulphadimidine | 53.3 | 53.3 | 53.3 | 53.3 | 53.3 | 53.3 |
| Iron Powder | 30.95 | 30.7 | 30.45 | 30.2 | 30.2 | 30.2 |
| Barium sulphate | 10.0 | 10.0 | 10.0 | 10.0 | 10.0 | 10.0 |
| HPMC (Methocel K15M) | 0.25 | 0.5 | 0.75 | 1.0 | - | - |
| HPMC (Methocel K100m) | - | - | - | - | 1.0 | - |
| HPMC (Methocel E4M) | - | - | - | - | - | 1.0 |
| PVP K 30* | 5.0 | 5.0 | 5.0 | 5.0 | 5.0 | 5.0 |
| Magnesium stearate | 0.5 | 0.5 | 0.5 | 0.5 | 0.5 | 0.5 |

| | | | | | | |
|---|---|---|---|---|---|---|
| *2.5% w/w of PVP was added as a powder and 2.5% w/w as a solution having the following composition:- | | | | | | |

| | % w/w |
|---|---|
| PVP K30 | 23.8 |
| Distilled water | 38.1 |
| Industrial methylated spirits | 38.1 |

### Method

All the ingredients apart from the magnesium stearate were granulated. The magnesium stearate was then added to the granulate and the mixture compressed using a Manesty 35T compressing machine, to give boluses of the desired weight.

### Measurement of Hardness

The hardness of the unit dose forms prepared in accordance with the present invention was measured using a T30K Tensometer (J.J. Lloyd), which was modified so as to locate the dosage form in a 3-point pivot. The dosage form e.g. a bolus is supported on two lower pins which move in an upward direction until the dosage form meets an upper pin located midway between the two lower pins. Pressure is applied until the dosage form breaks and the force required to reach this point is recorded.

### Serum profiles following oral administration to cattle

### Example 6

Four cattle (nos 1-4) each received by oral administration a single 30g bolus formulated according to Example 1, containing 5.33% w/w of Compound B and 47.97% w/w of sulphadimidine (SDD). A further four cattle Nos. 5-8) each received by oral administration a single 30g bolus formulated according to Example 4, containing 5.33% w/w of Compound B and 47.97% w/w of SDD. Blood samples were taken from each animal at the time of dosing and at 3, 5, 7, 12, 24, 31, 48, 54, 72, 78, 96, 102, 120 and 168 hours after administration. The concentrations of compound B and SDD in the serum were determined by bioassay and the Bratton-Marshall colorimetric method respectively.

The bodyweights of the cattle were as follows:

| Cattle No | Bodyweight (kg) | Cattle No | Bodyweight (kg) |
|---|---|---|---|
| 1 | 205 | 5 | 170 |
| 2 | 160 | 6 | 165 |
| 3 | 180 | 7 | 180 |
| 4 | 215 | 8 | 215 |

### Results

### Bolus of Example 1

| Time (hours) | Compound B µg/cm³ serum | | | | SDD µg/cm³ serum | | | |
|---|---|---|---|---|---|---|---|---|
| | Animal No. | | | | | | | |
| | 1 | 2 | 3 | 4 | 1 | 2 | 3 | 4 |
| 0 | 0 | 0 | 0 | 0 | 0 | 0 | 0 | 0 |
| 3 | 0.07 | 0.05 | 0.06 | 0.05 | 23 | 23 | 23 | 21 |
| 5 | 0.14 | 0.12 | 0.13 | 0.11 | 50 | 44 | 40 | 39 |
| 7 | 0.27 | 0.23 | 0.23 | 0.19 | 65 | 64 | 59 | 50 |
| 12 | 0.43 | 0.49 | 0.47 | 0.46 | 72 | 89 | 78 | 63 |
| 24 | 0.75 | 0.98 | 0.85 | 0.68 | 38 | 68 | 44 | 37 |
| 27 | 1.60 | 1.80 | 1.80 | 1.10 | 28 | 53 | 38 | 27 |
| 31 | 1.40 | 1.30 | 1.40 | 0.82 | 21 | 36 | 30 | 19 |
| 48 | 0.57 | 0.62 | 0.63 | 0.49 | 2 | 8 | 7 | 3 |
| 54 | 0.43 | 0.43 | 0.50 | 0.37 | 0 | 5 | 3 | 2 |
| 72 | 0.18 | 0.22 | 0.23 | 0.16 | 0 | 1 | 1 | 1 |
| 78 | 0.13 | 0.13 | 0.18 | 0.13 | 0 | 0 | 0 | 0 |
| 96 | 0.05 | 0.06 | 0.10 | 0.05 | 0 | 0 | 0 | 0 |
| 102 | 0 | 0 | 0.06 | 0 | 0 | 0 | 0 | 0 |
| 120 | 0 | 0 | 0 | 0 | 0 | 0 | 0 | 0 |
| 168 | 0 | 0 | 0 | 0 | 0 | 0 | 0 | 0 |

### Bolus of Example 4

| Time (hours) | Compound B µg/cm³ serum | | | | SDD µg/cm³ serum | | | |
|---|---|---|---|---|---|---|---|---|
| | Animal No. | | | | | | | |
| | 5 | 6 | 7 | 8 | 5 | 6 | 7 | 8 |
| 0 | 0 | 0 | 0 | 0 | 0 | 0 | 0 | 0 |
| 3 | 0 | 0 | 0 | 0 | 1.5 | 2.5 | 2 | 2 |
| 5 | 0 | 0 | 0 | 0 | 2 | 5 | 4.5 | 4 |
| 7 | 0 | 0 | 0 | 0 | 3.5 | 7 | 7.5 | 9 |
| 12 | 0 | 0 | 0.06 | 0 | 6 | 9 | 12.5 | 9 |
| 24 | 0.17 | 0.15 | 0.22 | 0.14 | 11.5 | 16.5 | 24.5 | 16.5 |
| 27 | 0.27 | 0.24 | 0.41 | 0.23 | 11.5 | 14 | 24.5 | 15 |
| 31 | 0.27 | 0.26 | 0.54 | 0.32 | 12.5 | 15.5 | 23.5 | 16 |
| 48 | 0.37 | 0.30 | 0.67 | 0.46 | 11 | 12.5 | 28 | 12.5 |
| 54 | 0.32 | 0.29 | 0.67 | 0.36 | 12 | 12.5 | 19 | 14.5 |
| 72 | 0.37 | 0.25 | 0.67 | 0.38 | 9.5 | 11.5 | 19 | 11 |
| 78 | 0.29 | 0.26 | 0.60 | 0.36 | 10 | 9 | 10 | 10.5 |
| 96 | 0.29 | 0.21 | 0.35 | 0.39 | 6.5 | 8 | 1.5 | 7.5 |
| 102 | 0.24 | 0.20 | 0.26 | 0.35 | 6 | 8 | 1 | 7 |
| 120 | 0.22 | 0.17 | 0.14 | 0.21 | 5 | 7 | 1 | 5.5 |
| 168 | 0.13 | 0.10 | 0 | 0 | 4.5 | 5.5 | 0.5 | 1.5 |

## Claims (Claims for the following Contracting State(s): DE, GB, FR, IT, NL, SE, LI, CH, BE, LU)

1. A veterinary composition for release of a biologically active substance into the rumen of an animal, which composition comprises said biologically active substance and a water swellable polymer material, characterised in that the water swellable polymer material is hydroxypropylmethylcellulose and constitutes from 0.01% to 4% by weight of the said composition.

2. A composition according to claim 1, in unit dose form.

3. A composition according to either of claims 1 or 2 adapted for oral administration to the rumen of an animal.

4. A composition according to claim 3 which is an intra-rumenal bolus provided with a means to inhibit regurgitation.

5. A composition according to claim 1 wherein the hydroxypropylmethylcellulose content is from 0.1 to 4.0% by weight of the total composition.

6. A composition according to claim 5 wherein the hydroxypropylmethylcellulose content is from 0.5 to 2.5% by weight of the total composition.

7. A composition according to any of claims 1 to 6 wherein the hydroxypropylmethylcellulose constitutes no more than 2.0% by weight of said composition.

8. A composition according to claim 7 wherein the hydroxypropylmethylcellulose content is between 0.1 and 1.0% by weight of the total composition.

9. A composition according to any of claims 1 to 8 wherein the biologically active substance is an antibacterial or anthelmintic agent.

10. A composition according to claim 9 wherein the antibacterial agent comprises a sulphonamide or a salt thereof and/or a 2,4-diaminopyrimidine or a salt thereof.

11. A composition according to claim 10 wherein the antibacterial agent comprises 2,4-diamino-5-(8-dimethylamino-7-methyl-5-quinolylmethyl) pyrimidine or a salt thereof together with sulphadimidine or a salt thereof.

12. A composition according to claim 11 which provides a dose of 2,4-diamino-5-(8-dimethylamino-7-methyl-5-quinolylmethyl) pyrimidine in the range 1 to 16mg/kg and a dose of sulphadimidine in the range 9 to 144mg/kg.

13. A composition according to claim 12 which is a veterinary bolus for oral administration to the rumen of an animal, and which provides a dose of 2,4-diamino-5-(8-dimethylamino-7-methyl-5-quinolylmethyl)-pyrimdine in the range from 1 to 8mg/kg and a dose of sulphadimidine in the range 9 to 72mg/kg and contains from 1 to 2% of hydroxypropylmethylcellulose by weight of the total composition.

14. A bolus according to claim 13 wherein the dose of the pyrimidine is 4mg/kg and the dose of sulphadimidine is 36mg/kg.

15. A composition according to claim 12 which is a veterinary bolus for oral administration to the rumen of an animal and which provides a dose of 2,4-diamino-5-(8-dimethylamino-7-methyl-5-quinolylmethyl)-pyrimidinein the range 6 to 16mg/kg and a dose of sulphadimidine in the range 54 to 144mg/kg and contains from 0.1 to 1.0% of hydroxypropylmethylcellulose by weight of the total composition.

16. A composition according to claim 15 wherein the dose of the pyrimidine is 8mg/kg and the case of sulphadimidine is 72mg/kg.

## Claims (Claims for the following Contracting State(s): AT, ES, GR)

1. A method for the preparation of a veterinary composition for release of a biologically active substance into the rumen of an animal, which comprises bringing into association said biologically active substances and a water swellable polymer material, characterised in that the water swellable polymer material is hydroxypropylmethylcellulose and constitutes from 0.01% to 4% by weight of the said composition.

2. A method according to claim 1 wherein the composition is in unit dose form.

3. A method according to either of claims 1 or 2 wherein the composition is adapted for oral administration to the rumen of an animal.

4. A method according to claim 3 wherein the composition is an intra-rumenal bolus provided with a means to inhibit regurgitation.

5. A method according to claim 1 wherein the hydroxypropylmethylcellulose content is from 0.1 to 4.0% by weight of the total composition.

6. A method according to claim 5 wherein the hydroxypropylmethylcellulose content is from 0.5 to 2.5% by weight of the total composition.

7. A method according to any of claims 1 to 6 wherein the hydroxypropylmethylcellulose constitutes no more than 2.0% by weight of said composition.

8. A method according to claim 7 wherein the hydroxypropylmethylcellulose content is between 0.1 and 1.0% by weight of the total composition.

9. A method according to any of claims 1 to 8 wherein the biologically active substance is an antibacterial or anthelmintic agent.

10. A method according to claim 9 wherein the antibacterial agent comprises a sulphonamide or a salt thereof and/or a 2,4-diaminopyrimidine or a salt thereof.

11. A method according to claim 10 wherein the antibacterial agent comprises 2,4-diamino-5-(8-dimethylamino-7-methyl-5-quinolylmethyl) pyrimidine or a salt thereof together with sulphadimidine or a salt thereof.

12. A method according to claim 11 wherein the composition provides a dose of 2,4-diamino-5-(8-dimethylamino-7-methyl-5-quinolylmethyl) pyrimidine in the range 1 to 16mg/kg and a dose of sulphadimidine in the range 9 to 144mg/kg.

13. A method according to claim 12 wherein the composition is a veterinary bolus for oral administration to the rumen of an animal and which provides a dose of 2,4-diamino-5-(8-dimethylamino-7-methyl-5-quinolylmethyl)pyrimidine in the range from 1 to 8mg/kg and a dose of sulphadimidine in the range 9 to 72mg/kg and contains from 1 to 2% of hydroxypropylmethylcellulose by weight of the total composition.

14. A method according to claim 13 wherein the dose of the pyrimidine is 4mg/kg and the dose of sulphadimidine is 36mg/kg.

15. A method according to claim 12 wherein the composition is a veterinary bolus for oral administration to the rumen of an animal and which provides a dose of 2,4-diamino-5-(8-dimethylamino-7-methy-5-quinolylmethyl)pyrimidine in the range 6 to 16mg/kg and a dose of sulphadimidine in the range 54 to 144mg/kg and contains from 0.1 to 1.0% of hydroxypropylmethylcellulose by weight of the total composition.

16. A method according to claim 15 wherein the dose of the pyrimidine is 8mg/kg and the dose of sulphadimidine is 72mg/kg.

## Patentansprüche (Patentansprüche für folgende(n) Vertragsstaat(en): DE, GB, FR, IT, NL, SE, LI, CH, BE, LU)

1. Veterinärmedizinische Zusammensetzung zur Freisetzung einer biologisch aktiven Substanz in das Rumen eines Tieres, wobei die Zusammensetzung die biologisch aktive Substanz und ein in Wasser quellbares Polymermaterial umfaßt, dadurch gekennzeichnet, daß das in Wasser quellbare Polymermaterial Hydroxypropylmethylcellulose ist und 0,01 bis 4 Gew.-% der Zusammensetzung ausmacht.

2. Zusammensetzung nach Anspruch 1 in Einheitsdosisform.

3. Zusammensetzung nach Anspruch 1 oder 2 zur oralen Verabreichung in das Rumen eines Tieres.

4. Zusammensetzung nach Anspruch 3, bei der es sich um einen intraruminalen Bolus handelt, der ein Mittel zur Verhinderung der Regurgitation aufweist.

5. Zusammensetzung nach Anspruch 1, worin die Hydroxypropylmethylcellulose in einer Menge von 0,1 bis 4,0 Gew.-%, bezogen auf die Gesamtzusammensetzung, enthalten ist.

6. Zusammensetzung nach Anspruch 5, worin die Hydroxypropylmethylcellulose in einer Menge von 0,5 bis 2,5 Gew.-%, bezogen auf die Gesamtzusammensetzung, enthalten ist.

7. Zusammensetzung nach einem der Ansprüche 1 bis 6, worin die Hydroxypropylmethylcellulose nicht mehr als 2,0 Gew.-% der Zusammensetzung ausmacht.

8. Zusammensetzung nach Anspruch 7, worin die Hydroxypropylmethylcellulose in einer Menge von 0,1 bis 1,0 Gew.-%, bezogen auf die Gesamtzusammensetzung, enthalten ist.

9. Zusammensetzung nach einem der Ansprüche 1 bis 8, worin die biologisch aktive Substanz ein antibakteriellas oder anthelmintisches Mittel ist.

10. Zusammensetzung nach Anspruch 9, worin das antibakterielle Mittel ein Sulfonamid oder ein Salz davon und/oder ein 2,4-Diaminopyrimidin oder ein Salz davon umfaßt.

11. Zusammensetzung nach Anspruch 10, worin das antibakterielle Mittel 2,4-Diamino-5-(8-dimethylamino-7-methyl-5-chinolylmethyl)pyrimidin oder ein Salz davon, zusammen mit Sulfamidin oder einem Salz davon, umfaßt.

12. Zusammensetzung nach Anspruch 11, welche eine 2,4-Diamino-5-(8-dimethylamino-7-methyl-5-chinolylmethyl)pyrimidin-Dosis im Bereich von 1 bis 16 mg/kg und eine Sulfamidin-Dosis im Bereich von 9 bis 144 mg/kg bereitstellt.

13. Zusammensetzung nach Anspruch 12, bei welcher es sich um einen veterinärmedizinischen Bolus zur oralen Verabreichung in das Rumen eines Tieres handelt und welche eine 2,4-Diamino-5-(8-dimethylamino-7-methyl-5-chinolylmethyl)pyrimidin-Dosis im Bereich von 1 bis 8 mg/kg und eine Sulfamidin-Dosis im Bereich von 9 bis 72 mg/kg bereitstellt und Hydroxypropylmethylcellulose in einer Menge von 1 bis 2 Gew.-%, bezogen auf das Gesamtgewicht der Zusammensetzung, enthält.

14. Bolus nach Anspruch 13, worin die Pyrimidin-Dosis 4 mg/kg und die Sulfamidin-Dosis 36 mg/kg beträgt.

15. Zusammensetzung nach Anspruch 12, bei welcher es sich um einen veterinärmedizinischen Bolus zur oralen Verabreichung in das Rumen eines Tieres handelt und welche eine 2,4-Diamino-5-(8-dimethylamino-7-methyl-5-chinolylmethyl)pyrimidin-Dosis im Bereich von 6 bis 16 mg/kg und eine Sulfamidin-Dosis im Bereich von 54 bis 144 mg/kg bereitstellt und Hydroxypropylmethylcellulose in einer Menge von 0,1 bis 1,0 Gew.-%, bezogen auf das Gesamtgewicht der Zusammensetzung, enthält.

16. Zusammensetzung nach Anspruch 15, worin die Pyrimidin-Dosis 8 mg/kg und die Sulfamidin-Dosis 72 mg/kg beträgt.

## Patentansprüche (Patentansprüche für folgende(n) Vertragsstaat(en): AT, ES, GR)

1. Verfahren zur Herstellung einer veterinärmedizinischen Zusammensetzung zur Freisetzung einer biologisch aktiven Substanz in das Rumen eines Tieres, wobei die Zusammensetzung die biologisch aktive Substanz und ein in Wasser quellbares Polymermaterial umfaßt, dadurch gekennzeichnet, daß das in Wasser quellbare Polymermaterial Hydroxypropylmethylcellulose ist und 0,01 bis 4 Gew.-% der Zusammensetzung ausmacht.

2. Verfahren nach Anspruch 1, wobei die Zusammensetzung in Einheitsdosisform vorliegt.

3. Verfahren nach Anspruch 1 oder 2, wobei die Zusammensetzung zur oralen Verabreichung in das Rumen eines Tieres bestimmt ist.

4. Verfahren nach Anspruch 3, wobei es sich bei der Zusammensetzung um einen intraruminalen Bolus handelt, der ein Mittel zur Verhinderung der Regurgitation aufweist.

5. Verfahren nach Anspruch 1, wobei die Hydroxypropylmethylcellulose in einer Menge von 0,1 bis 4,0 Gew.-%, bezogen auf die Gesamtzusammensetzung, enthalten ist.

6. Verfahren nach Anspruch 5, wobei die Hydroxypropylmethylcellulose in einer Menge von 0,5 bis 2,5 Gew.-%, bezogen auf die Gesamtzusammensetzung, enthalten ist.

7. Verfahren nach einem der Ansprüche 1 bis 6, wobei die Hydroxypropylmethylcellulose nicht mehr als 2,0 Gew.-% der Zusammensetzung ausmacht.

8. Verfahren nach Anspruch 7, wobei die Hydroxypropylmethylcellulose in einer Menge von 0,1 bis 1,0 Gew.-%, bezogen auf die Gesamtzusammensetzung, enthalten ist.

9. Verfahren nach einem der Ansprüche 1 bis 8, wobei die biologisch aktive Substanz ein antibakterielles oder anthelmintisches Mittel ist.

10. Verfahren nach Anspruch 9, wobei das antibakterielle Mittel ein Sulfonamid oder ein Salz davon und/oder ein 2,4-Diaminopyrimidin oder ein Salz davon umfaßt.

11. Verfahren nach Anspruch 10, wobei das antibakterielle Mittel 2,4-Diamino-5-(8-dimethylamino-7-methyl-5-chinolylmethyl)pyrimidin oder ein Salz davon, zusammen mit Sulfamidin oder einem Salz davon, umfaßt.

12. Verfahren nach Anspruch 11, wobei die Zusammensetzung eine 2,4-Diamino-5-(8-dimethylamino-7-methyl-5-chinolylmethyl)pyrimidin-Dosis im Bereich von 1 bis 16 mg/kg und eine Sulfamidin-Dosis im Bereich von 9 bis 144 mg/kg bereitstellt.

13. Verfahren nach Anspruch 12, wobei es sich bei der Zusammensetzung um einen veterinärmedizinischen Bolus zur oralen Verabreichung in das Rumen eines Tieres handelt und welche eine 2,4-Diamino-5-(8-dimethylamino-7-methyl-5-chinolylmethyl)pyrimidin-Dosis im Bereich von 1 bis 8 mg/kg und eine Sulfamidin-Dosis im Bereich von 9 bis 72 mg/kg bereitstellt und Hydroxypropylmethylcellulose in einer Menge von 1 bis 2 Gew.-%, bezogen auf das Gesamtgewicht der Zusammensetzung, enthält.

14. Verfahren nach Anspruch 13, wobei die Pyrimidin-Dosis 4 mg/kg und die Sulfamidin-Dosis 36 mg/kg beträgt.

15. Verfahren nach Anspruch 12, wobei es sich bei der Zusammensetzung um einen veterinärmedizinischen Bolus zur oralen Verabreichung in das Rumen eines Tieres handelt und welche eine 2,4-Diamino-5-(8-dimethylamino-7-methyl-5-chinolylmethyl)pyrimidin-Dosis im Bereich von 6 bis 16 mg/kg und eine Sulfamidin-Dosis im Bereich von 54 bis 144 mg/kg bereitstellt und Hydroxypropylmethylcellulose in einer Menge von 0,1 bis 1,0 Gew.-%, bezogen auf das Gesamtgewicht der Zusammensetzung, enthält.

16. Verfahren nach Anspruch 15, wobei die Pyrimidin-Dosis 8 mg/kg und die Sulfamidin-Dosis 72 mg/kg beträgt.

## Revendications (Revendications pour l'(les) Etat(s) contractant(s) suivant(s): DE, GB, FR, IT, NL, SE, LI, CH, BE, LU)

1. Composition vétérinaire destinée à libérer une substance biologiquement active dans le rumen d'un animal, laquelle composition renferme ladite substance biologiquement active et un matériau polymère gonflant dans l'eau, caractérisée en ce que le matériau polymère gonflant dans l'eau est l'hydroxypropylméthylcellulose et représente de 0,01% à 4% en poids, par rapport au poids de ladite composition.

2. Composition selon la revendication 1, sous forme de dose unitaire.

3. Composition selon l'une des revendications 1 ou 2, adaptée à l'administration orale dans le rumen d'un animal.

4. Composition selon la revendication 3, qui est un bol, destiné à parvenir à l'intérieur du rumen, équipé d'un agent de rétention pour empêcher la régurgitation.

5. Composition selon la revendication 1, dans laquelle la proportion d'hydroxypropylméthylcellulose est comprise entre 0,1 et 4,0% en poids, par rapport au poids total de la composition.

6. Composition selon la revendication 5, dans laquelle la proportion d'hydroxypropylméthylcellulose est comprise entre 0,5 et 2,5% en poids, par rapport au poids total de la composition.

7. Composition selon l'une quelconque des revendications 1 à 6, dans laquelle l'hydroxypropylméthylcellulose ne représente pas plus de 2% en poids de ladite composition.

8. Composition selon la revendication 8, dans laquelle la proprotion d'hydroxypropylméthylcellulose est comprise entre 0,1 et 1,0% en poids, par rapport au poids total de la composition.

9. Composition selon l'une quelconque des revendications 1 à 8, dans laquelle la substance biologiquement active est un agent antibactérien ou anti-helminthique.

10. Composition selon la revendication 9, dans laquelle l'agent antibactérien comporte un sulfamide, ou un sel de celui-ci, et/ou une 2,4-diaminopyrimidine, ou un sel de celle-ci.

11. Composition selon la revendication 10, dans laquelle l'agent antibactérien comporte de la 2,4-diamino-5-(8-diméthylamino-7-méthyl-5-quinolylméthyl)-pyrimidine, ou un sel de celle-ci, conjointement avec de la sulfadimidine, ou un sel de celle-ci.

12. Composition selon la revendication 11, où la composition fournit une dose de 2,4-diamino-5-(8-diméthylamino-7-méthyl-5-quinolylméthyl)-pyrimidine, située dans le domaine allant de 1 à 16 mg/kg, et une dose de sulfadimidine, située dans le domaine allant de 9 à 144 mg/kg.

13. Composition selon la revendication 12, où la composition est un bol vétérinaire, destiné à l'administration orale dans le rumen d'un animal, et qui fournit une dose de 2,4-diamino-5-(8-diméthylamino-7-méthyl-5-quinolylméthyl)-pyrimidine, située dans le domaine allant de 1 à 8 mg/kg, et une dose de sulfadimidine, située dans le domaine allant de 9 à 72 mg/kg, et contient de 1 à 2% en poids d'hydroxypropylméthylcellulose, par rapport au poids total de la composition.

14. Bol selon la revendication 13, dans laquelle la dose de pyrimidine est de 4 mg/kg et la dose de sulfadimidine est de 36 mg/kg.

15. Composition selon la revendication 12, qui est un bol vétérinaire, destiné à l'administration orale dans le rumen d'un animal, et qui fournit une dose de 2,4-diamino-5-(8-diméthylamino-7-méthyl-5-quinolylméthyl)-pyrimidine, située dans le domaine allant de 6 à 16 mg/kg, et une dose de sulfadimidine, située dans le domaine allant de 54 à 144 mg/kg, et contient de 0,1 à 1,0 % en poids d'hydroxypropylméthylcellulose, par rapport au poids total de la composition.

16. Méthode selon la revendication 15, dans laquelle la dose de pyrimidine est de 8 mg/kg et la dose de sulfadimidine est de 72 mg/kg.

## Revendications (Revendications pour l'(les) Etat(s) contractant(s) suivant(s): AT, ES, GR)

1. Méthode de préparation d'une composition vétérinaire destinée à libérer une substance biologiquement active dans le rumen d'un animal, comprenant l'association de ladite substance biologiquement active à un matériau polymère gonflant dans l'eau, caractérisée en ce que le matériau polymère gonflant dans l'eau est l'hydroxypropylméthylcellulose et représente de 0,01% à 4% en poids, par rapport au poids de ladite composition.

2. Méthode selon la revendication 1, où la composition est sous forme de dose unitaire.

3. Méthode selon l'une des revendications 1 ou 2, où la composition est adaptée à l'administration orale dans le rumen d'un animal.

4. Méthode selon la revendication 3, où la composition est un bol, destiné à parvenir à l'intérieur du rumen, équipé d'un agent de rétention pour empêcher la régurgitation.

5. Méthode selon la revendication 1, dans laquelle la proportion d'hydroxypropylméthylcellulose est comprise entre 0,1 et 4,0% en poids, par rapport au poids total de la composition.

6. Méthode selon la revendication 5, dans laquelle la proportion d'hydroxypropylméthylcellulose est comprise entre 0,5 et 2,5% en poids, par rapport au poids total de la composition.

7. Méthode selon l'une quelconque des revendications 1 à 6, dans laquelle l'hydroxypropylméthylcellulose ne représente pas plus de 2% en poids de ladite composition.

8. Méthode selon la revendication 7, dans laquelle la proprotion d'hydroxypropylméthylcellulose est comprise entre 0,1 et 1,0% en poids, par rapport au poids total de la composition.

9. Méthode selon l'une quelconque des revendications 1 à 8, dans laquelle la substance biologiquement active est un agent antibactérien ou anti-helminthique.

10. Méthode selon la revendication 9, dans laquelle l'agent antibactérien comporte un sulfamide, ou un sel de celui-ci, et/ou une 2,4-diaminopyrimidine, ou un sel de celle-ci.

11. Méthode selon la revendication 10, dans laquelle l'agent antibactérien comporte de la 2,4-diamino-5-(8-diméthylamino-7-méthyl-5-quinolylméthyl)-pyrimidine, ou un sel de celle-ci, conjointement avec de la sulfadimidine, ou un sel de celle-ci.

12. Méthode selon la revendication 11, où la composition fournit une dose de 2,4-diamino-5-(8-diméthylamino-7-méthyl-5-quinolylméthyl)-pyrimidine, située dans le domaine allant de 1 à 16 mg/kg, et une dose de sulfadimidine, située dans le domaine allant de 9 à 144 mg/kg.

13. Méthode selon la revendication 12, où la composition est un bol vétérinaire, destiné à l'administration orale dans le rumen d'un animal, et qui fournit une dose de 2,4-diamino-5-(8-diméthylamino-7-méthyl-5-quinolylméthyl)-pyrimidine, située dans le domaine allant de 1 à 8 mg/kg, et une dose de sulfadimidine, située dans le domaine allant de 9 à 72 mg/kg, et contient de 1 à 2% en poids d'hydroxypropylméthylcellulose, par rapport au poids total de la composition.

14. Méthode selon la revendication 13, dans laquelle la dose de pyrimidine est de 4 mg/kg et la dose de sulfadimidine est de 36 mg/kg.

15. Méthode selon la revendication 12, où la composition vétérinaire, destiné à l'administration orale dans le rumen d'un animal, et qui fournit une dose de 2,4-diamino-5-(8-diméthylamino-7-méthyl-5-quinolylméthyl)-pyrimidine, située dans le domaine allant de 6 à 16 mg/kg, et une dose de sulfadimidine, située dans le domaine allant de 54 à 144 mg/kg, et contient de 0,1 à 1,0 % en poids d'hydroxypropylméthylcellulose, par rapport au poids total de la composition.

16. Méthode selon la revendication 15, dans laquelle la dose de pyrimidine est de 8 mg/kg et la dose de sulfadimidine est de 72 mg/kg.
